(19) Europäisches Patentamt
European Patent Office
Office européen des brevets

(11) EP 1 118 615 A1

(12) EUROPEAN PATENT APPLICATION
published in accordance with Art. 158(3) EPC

(43) Date of publication:
**25.07.2001 Bulletin 2001/30**

(21) Application number: **99944776.6**

(22) Date of filing: **22.09.1999**

(51) Int Cl.[7]: **C07D 471/04**, A61K 31/4985, A61K 45/00, A61P 43/00, A61P 1/16, A61P 11/00, A61P 11/06, A61P 27/02, A61P 27/06, A61P 27/14, A61P 31/18, A61P 37/08

(86) International application number:
**PCT/JP99/05182**

(87) International publication number:
**WO 00/18768 (06.04.2000 Gazette 2000/14)**

(84) Designated Contracting States:
**AT BE CH CY DE DK ES FI FR GB GR IE IT LI LU MC NL PT SE**
Designated Extension States:
**AL LT LV MK RO SI**

(30) Priority: **29.09.1998 JP 27444098**

(71) Applicant: **FUJISAWA PHARMACEUTICAL CO., LTD.**
**Osaka-shi Osaka 541-8514 (JP)**

(72) Inventors:
- **SHIMAZAKI, Norihiko**
  **Ibaraki 300-0817 (JP)**
- **WATANABE, Masaru**
  **Hyogo 663-8103 (JP)**
- **ICHIHARA, Masaharu**
  **Osaka 562-0003 (JP)**
- **KAWAI, Nobutaka**
  **Osaka 552-0014 (JP)**
- **MACHIYA, Koji**
  **Hyogo 657-0028 (JP)**
- **KAGARA, Kooji**
  **Osaka 562-0032 (JP)**

(74) Representative:
**Gille Hrabal Struck Neidlein Prop Roos**
**Patentanwälte**
**Brucknerstrasse 20**
**40593 Düsseldorf (DE)**

# (54) NOVEL SALTS OF PYRIDOPYRAZINE COMPOUND AND CRYSTALS THEREOF

(57) The invention provides a novel pyridopyrazine compound salt of the following formula and its crystal, which are very satisfactory in solubility and stability.

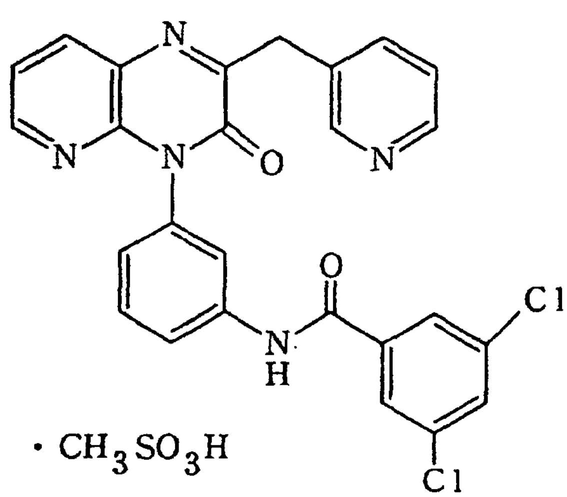

• $CH_3SO_3H$

(I)

EP 1 118 615 A1

More particularly, the compound of the above formula, its solvate and their crystals are provided.

## Description

TECHNICAL FIELD

This invention relates to the methanesulfonate, which is novel, of a pyridopyrazine compound of use as a medicament. More particularly, this invention is concerned with providing 4-[3-(3,5-dichlorobenzoylamino)phenyl]-2-(3-pyridylmethyl)-3-oxo-3,4-dihydropyr ido[2,3-b]pyrazine methanesulfonate of the following formula (I) [hereinafter referred to as compound (I)], a solvate (hydrate, ethanolate, etc.) thereof, their crystals and novel pharmaceutical uses thereof.

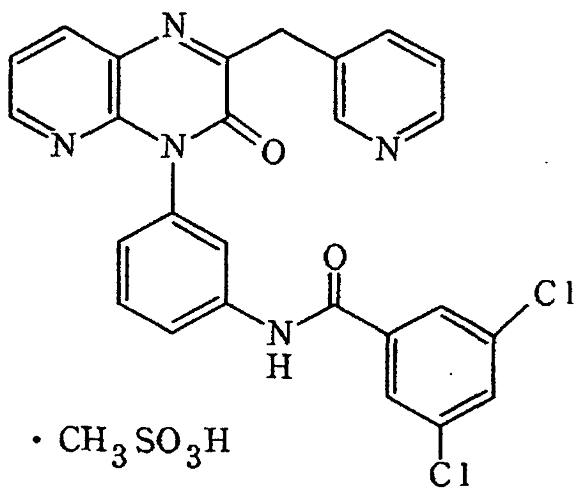

(I)

The pyridopyrazine compound of formula (A), namely 4-[3-(3,5-dichlorobenzoylamino)phenyl]-2-(3-pyridylmethyl)-3-oxo-3,4-dihydropyr ido[2,3-b]pyrazine [hereinafter referred to as free base (A)] and its hydrochloride [hereinafter referred to as hydrochloride (B)] are described in PCT laid-open specification WO 96/01825 and, as such, are known.

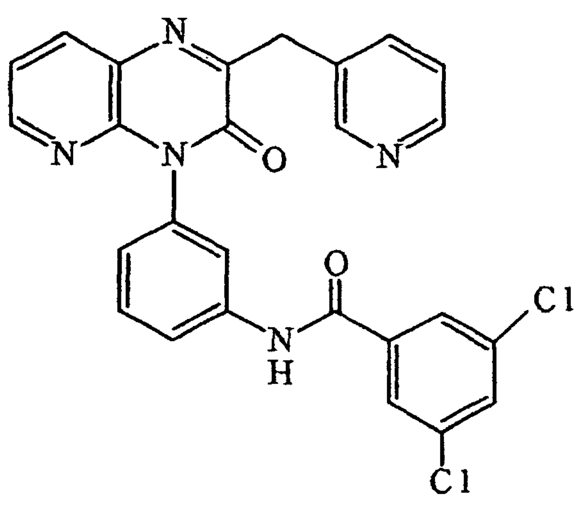

(A)

DISCLOSURE OF INVENTION

Freebase (A) cannot be easily dissolved in water in pharmaceutical production, for instance, and hydrochloride (B) is not as stable as desired so that improvements in solubility and stability have been needed.

The inventors of this invention explored into a variety of salts for enhancing the usefulness of free base (A) as a drug, that is to say improving the solubility and stability thereof, and as a result arrived at the compound (I) which

is the subject of the instant application. They further discovered that the compound (I) according to this invention has not only phosphodiesterase IV (PDE-IV) inhibitory activity and tumor necrosis factor (TNF α) production inhibitory activity of the same order as those of free base (A) but also interferon (INF γ) and interleukin (IL-2, IL-4, IL-5, IL-10, etc.) production inhibitory activity and, based on such activities, inhibits infiltration of various inflammatory cells such as macrophages, lymphocytes, eosinophils, and neutrophils at inflammation sites. This invention has come forth from the above finding.

The compound (I) of this invention is novel and has been improved in the solubility in distilled water for injection and physiological saline solution as compared with free base (A) and in the crystallinity and stability on compounding with ethanol as compared with hydrochloride (B).

Concerning compound (I), six different polymorphs have so far been identified, and the inventors established processes for producing the respective polymorphs and named those polymorphs the A01 crystal, A09 crystal, A16 crystal, A20 crystal, A30 crystal and A43 crystal, respectively. The identification of the respective crystals was based on the powder X-ray diffraction patterns (Fig. 1, Fig. 2, Fig. 3, Fig. 4, Fig. 5 and Fig. 6).

Of those crystals, the polymorph A20 crystal corresponds to the anhydride, the A30 crystal the hydrate-ethanolate, and the remaining crystals hydrates.

While those polymorphs are invariably superior to free base (A) and hydrochloride (B) in various characteristics as mentioned above, the most preferred is the polymorph A16 which, in the evaluation of stability, solubility and absorbability, was found to be least variable in crystal structure on standing in solid state or during compounding and has been most improved in solubility.

The A16 crystal substantially shows the powder X-ray diffraction characteristics given under Data 3. Diffraction data were generated with Philip's MPD1880 powder X-ray diffraction analyzer using monochromated CuK α radiation as X-rays, The following data 1~6, derived from Figs. 1~6, are respectively shown as rearranged in the increasing order of the 2θ value.

It is only sufficient that the polymorphs according to this invention substantially satisfy the corresponding diffraction patterns given under Data 1~6, that is to say no strict agreement is required.

Data 1: A01 crystal

2θ (° )=2.78, 5.59, 14.98, 16.73, 19.79, 24.61, 26.07

Data 2: A09 crystal

2θ (° )=7.72, 10.07, 13.69, 15.23, 20.26, 21.03, 24.74, 25.17

Data 3: A16 crystal

2θ (° )=2.86, 5.63, 20.86, 22.31, 23.88, 25.93

Data 4: A20 crystal

2θ (°)=12.50, 19.28, 21.50, 23.16, 25.38

Data 5: A30 crystal

2θ (°)=7.72, 10.03, 13.61, 20.05, 24.18

Data 6: A43 crystal

2θ (° )=7.08, 12.20, 21.33

The compound (I) of this invention has not only phosphodiesterase IV (PDE-IV) inhibitory activity and tumor necrosis factor (TNF α) production inhibitory activity of the same order as those of free base (A) but also interferon (INF γ) and interleukin (IL-2, IL-4, IL-5, IL-10, etc.) production inhibitory activities and, based on such activities, is capable of inhibiting infiltration of various inflammatory cells such as macrophages, lymphocytes, eosinophils, and neutrophils in foci of inflammation.

In the realm of hepatitis, because of its potent liver cell necrosis inhibitory activity and inflammatory cell infiltration inhibitory activity, the compound (I) of this invention is particularly useful for the treatment of viral [type A~E, type G, herpesvirus group (human herpesvirus 1 and 2, varicella-zoster virus, cytomegalovirus, Epstein-Barr (EB) virus, adenovirus, etc.), paramyxovirus, etc.], autoimmune or drug-induced (allergic) hepatitis, in the fulminating, acute

and chronic stages of liver impairment, and hepatitis in the fulminating, acute and chronic stages of liver impairment associated with poisoning (acetaminophen overdose-induced hepatotoxicity, Amanita mushroom poisoning, industrial solvent and other poisoning), ischemia (hepatic vein occlusion), hyperthermia, malignant cell infiltration of the liver, Wilson's disease, fatty liver, etc.

Furthermore, the compound (I) of this invention is not only effective in arresting the transition of various types of chronic hepatitis to cirrhosis or hepatocarcioma and in the treatment of primary biliary cirrhosis but is expected to be effective as a hepatoprotectant to be indicated postoperatively in liver transplantation or partial hepatectomy (in cases of liver transplantation, it is useful for premedication of donors as well).

In the department of ophthalmology, the compound (I) of this invention is expected to be effective in trachoma (Chlamydia trachomatis infection) in which TNF α is a suspected etiologic factor in tissue damage and cicatrization [Infection and Immunity, Vol.64 (1996), 3273-3279; ibid. Vol.65 (1997), 1003-1006], allergic (inflammatory and atopic) conjunctivitis in which infiltrating inflammatory cells, chiefly eosinophils, and inflammatory cytokines are suspected to play major roles, glaucoma in connection with which it is reported that the nonspecific phosphodiesterase inhibitor pentoxifylline administered topically lowers the rabbit intraocular pressure and that cAMP is an important factor in the regulation of intraocular pressure [European Journal of Pharmacology, Vol.258 (1994), 85-94], and ophthalmitis associated with a herpesvirus, HIV or other viral infection. In addition, the compound (I) is expected to be effective in the treatment of uveitis in connection with which anti-TNF α antibody and rolipram have been shown to be effective in the mouse model (experimental autoimmune uveoretinitis) [Investigative Ophthalmology & Visual Science, Vol.37 (1996), 2211-2218; ibid., Vol.40 (1999), 942-950].

Recent years have seen many reports indicating that TNF α and INF γ are playing essential roles in the proliferation of AIDS virus in immune cells, and with the aim to suppressing the production of TNF α, attempts have actually been made to administer pentoxyfylline to AIDS patients [Journal of Cardiovascular Pharmacology Vol.25 Suppl.2 (1995), S139-42]. Furthermore, it is reported that a PDE-IV inhibitor inhibits the HIV proliferation signal from cytokines [Journal of Virology, Vol.72 (1998), 4712-20]. The foregoing information suggests the possible efficacy of the compound (I) of this invention as a therapeutic drug for AIDS, too.

As to other indications, the demonstrated efficacy of free base (A) in the inhibition of eosinophil infiltration and in an animal model of delayed asthma suggest that the compound (I) is also effective in asthma and chronic obstructive pulmonary disease (COPD). The compound (I) is further useful for the treatment of atopic dermatitis.

As the compounds having phosphodiesterase IV (PDE-IV) inhibitory activity, tumor necrosis factor (TNF) production inhibitory activity, interferon production inhibitory activity and/or interleukin production inhibitory activity, all of which are considered to be useful activities for the above-mentioned applications, the following compounds and the compounds described in the United States Patents listed below can be mentioned in addition to the compound of the instant invention.

| Compound: | Manufacturer: |
|---|---|
| Arofylline | (Almirall-Prodesfarma), |
| Atizoram | (Pfizer), |
| AWD 12281 | (ASTA Medica), |
| BAY 198004 | (Bayer), |
| CDC 801 | (Celgene), |
| CDP 840 | (Celltech), |
| CI 1018 | (Parke-Davis), |
| Cipamfylline | (SmithKline Beecham), |
| CP 146523 | (Pfizer), |
| CP 166907 | (Pfizer), |
| CP 220629 | (Pfizer), |
| CP 293121 | (Pfizer), |
| CP 353164 | (Pfizer), |
| CP 77059 | (Pfizer), |
| CP 80633 | (Pfizer), |
| CT 1579 | (Merck Frosst), |
| CT 1786 | (Merck Frosst), |
| D 22888 | (Asta Medica), |
| D 4396 | (Chiroscience), |
| D 4418 | (Chiroscience), |

(continued)

| Compound: | Manufacturer: |
|---|---|
| DWP 205297 | (Daewoong), |
| Filaminast(PDA641) | (American Home Products), |
| GW 3600 | (Glaxo Wellcome), |
| KF 19514 | (Kyowa Hakko Kogyo), |
| L 0066 | (Pierre Fabre), |
| LAS 31025 | (Almirall-Prodesfarma), |
| LAS 32688 | (Almirall-Prodesfarma), |
| LAS 33774 | (Almirall-Prodesfarma), |
| MKS 213492 | (Novartis), |
| NCS 613, | |
| ORG 10325 | (Organon, Akzo Nobel), |
| ORG 30029 | (Organon, Akzo Nobel), |
| ORG 9731 | (Organon, Akzo Nobel), |
| PDB 093 | (Amerecan Home Products) , |
| PDE-IV inhibitors, Zambon | (Zambon), |
| Piclamilast(RP 73401) | (Rhone-Poulenc Rorer), |
| Roflumilast | (Byk Gulden), |
| Rolipram | (Shering AG), |
| RPR 116474 | (Rhone-Poulenc Rorer), |
| RPR 132294 | (Rhone-Poulenc Rorer), |
| RPR 132703 | (Rhone-Poulenc Rorer), |
| SB 207499 | (SmithKline Beecham), |
| SDZISQ 844 | (Novartis), |
| SelCIDs | (Celgene), |
| SH 636 | (Schering AG), |
| T 440 | (Tanabe Seiyaku), |
| Tolafentrine | (Byk Gulden, Altana), |
| WAY 122331 | (American Home Products), |
| WAY 127093B | (American Home Products), |
| WIN 65579 | (Sanofi Winthrop), |
| YM 976 | (Yamanouchi Pharmaceutical) |
| Zardaverine | (Byk Gulden, Altana) |

| United States Patent: | |
|---|---|
| USP | 3896000 |
| USP | 4356256 |
| USP | 5491147 |
| USP | 5580888 |
| USP | 5591776 |
| USP | 5622977 |
| USP | 5674880 |
| USP | 5686434 |
| USP | 5693659 |
| USP | 5710160 |
| USP | 5710170 |
| USP | 5712282 |
| USP | 5728712 |
| USP | 5744473 |

(continued)

| United States Patent: | |
|---|---|
| USP | 5747506 |
| USP | 5753666 |
| USP | 5773467 |
| USP | 5776958 |
| USP | 5780667, etc. |

The compound (I) of this invention has not only potent phosphodiesterase IV (PDE-IV) inhibitory activity and tumor necrosis factor (TNF) production inhibitory activity but also INF γ, IL-2, IL-4 and IL-5 production inhibitory activity (cf. the test methods A1~A3 described below) and is effective in the prophylaxis and/or therapy of various diseases. The usefulness of the compound (I) can be confirmed by the methods described below [cf. the test methods B1~B8], for instance.

Test methods A1~A3:

(A1) PDE-IV inhibitory activity

[Method] A crude enzyme was prepared from the cells (CHO cells) to which the cDNA for human PDE4-B1 isoform had been introduced for expression of the protein and the PDE4 inhibitory activity of the compound (I) of this invention was determined.

(A2) LPS-stimulated TNF α production inhibitory activity in human peripheral blood monocytes (PBMC)

[Method] Monocytes isolated from healthy adultmale volunteers were cultured and stimulated with LPS (1 g/ml) and the amount of TNF in the 24-hr culture supernatant was determined by ELISA.

(A3) Anti-CD3/CD28 antibody-stimulated TNF α production inhibitory activity in human peripheral blood monocytes (PBMC)

[Method] Monocytes isolated from healthy adult male volunteers were cultured and stimulated with anti-CD3 and anti-CD28 antibodies and the amounts of TNF, INF, 1L-2, IL-4 and IL-10 in the 24-hr culture supernatant were determined by ELISA.

Investigational substance:

The compound obtained in Example 2 (hereinafter referred to as the compound of this invention)

Results:

The results of tests A1~A3 are presented below.

| Test method | | $IC_{50}$ (M) |
|---|---|---|
| (A1) PDE-IV inhibitory activity | | $2.6x10^{-8}$ |
| (A2) LPS-stimulated TNF α production inhibitory activity in human peripheral blood monocytes (PBMC) | | $4.\ 82x10^{-9}$ |
| (A3) Anti-CD3/CD28 antibody-stimulated cytokine production inhibitory activity in human peripheral blood monocytes (PBMC) | TNF α | $4.45x10^{-8}$ |
| | TNF γ | $5.73x10^{-8}$ |
| | IL-2 | $1.30x10^{-7}$ |
| | IL-4 | $1.55x10^{-7}$ |
| | IL-10 | $5.97x10^{-8}$ |

Test Methods B1~B8

(B1) DGalN/LPS-induced rat hepatitis model

[Method] Male Wistar rats aged 12 weeks were deprived of food for 24 hours and dosed orally with the drug or the solvent vehicle in predetermined doses. Immediately thereafter, saline was administered from the tail vein to the NORMAL group and D-galactosamine hydrochloride (DGalN) (300 mg/kg)/LPS (0.32 µg/kg) was administered similarly to the CONTROL and DRUG groups. At 24 hr after administration of DGaIN/LAS, the blood was drawn and the liver isolated. Using the blood, liver-derived enzymes (ALT and AST) in plasma were assayed. The liver was fixed in formalin and phathological specimens were prepared. Histopathological findings (inflammatory cell infiltration and liver cell necrosis) were evaluated.

[Results] The compound of this invention significantly inhibited elevation of blood ALT and AST in the model used. Furthermore, the compound inhibited liver cell necrosis and inflammatory cell infiltration as well.

(B2) DGalN-induced rat hepatitis model

[Method] Male Wistar rats aged 12 weeks were deprived of food for 24 hours and dosed orally with the drug or the solvent vehicle in predetermined doses. Immediately thereafter, saline was administered intraperitoneally to the NORMAL group and D-galactosamine hydrochloride (DGalN), 400 mg/kg, was administered similarly to the CONTROL and DRUG groups. At 24 hours after administration of DGalN, the blood was drawn and the liver isolated. Using the blood, the liver-derived en zymes (ALT, AST) in plasma were assayed. The liver was fixed in formalin and pathological specimens were prepared. Histopathological findings (inflammatory cell infiltration and liver cell necrosis) were evaluated.

[Results] The compound of this invention significantly inhibited elevation of blood ALT and AST in this model. Furthermore, it inhibited liver cell necrosis and inflammatory cell infiltration as well.

(B3) ConA (concanavalin A) -induced mouse hepatitis model

[Method] Male Balb/c mice aged 9 weeks were deprived of food for 20 hours and the drug or the solvent vehicle was administered orally in predetermined doses. After 1 hour, saline was administered intravenously to the NORMAL group and concanavalin A, 0.4 mg/mouse, was administered similarly to the CONTROL and DRUG groups. At 18 hours after administration of ConA, the blood was drawn and the liver isolated. Using the blood, the liver-derived enzymes (ALT and AST) in plasma were assayed. The liver was fixed in formalin and pathological specimens were prepared. Histopathological findings (inflammatory cell infiltration and liver cell necrosis) were evaluated.

[Results] The compound of this invention significantly inhibited elevation of blood ALT and AST in this model. Furthermore, it inhibited liver cell necrosis and inflammatory cell infiltration as well.

(B4) P. acnes/LPS-induced mouse hepatitis model

[Method] Propionibacterium acnes (P. acnes) killed by heat treatment was lyophilized and used in the experiment. To 7-week-old male ICR mice, either saline or P. acnes, 0.6 mg/0.2 ml/mouse, was administered intravenously. After 7 days, the drug or the solvent vehicle was administered orally in predetermined doses to the animals deprived of food for 20 hours. At. 9 hours after administration of the drug, the blood was drawn and the liver-derived enzymes (ALT and AST) in plasma were assayed.

[Results] The compound of this invention significantly inhibited elevation of blood ALT and AST in this model.

(B5) LEC rat spontaneous hepatitis model

Long Evans Cinnamon (LEC) rats are known to spontaneously develop acute hepatitis with jaundice as a cardinal sign and 50~70% of them experience a fulminating stage and die. It is also known that almost all surviving animals run a clinical course of acute hepatitis-chronic hepatitis-biliary fibrosis (cirrhosis)-hepatocarcinoma.

[Method] To 9-week-old male LEC rats, either the drug or the solvent vehicle was administered orally in predetermined doses once daily for 23 consecutive weeks. On the day following the last dosing day, the blood was drawn and the liver isolated. Using the blood, the liver-derived enzymes (ALT, AST) in plasma were assayed. The liver was fixed in formalin and pathological specimens were prepared. Histopathological findings (inflammatory cell infiltration, biliary hyperplasia and liver cell necrosis) were evaluated.

[Results] The compound of this invention significantly inhibited the elevation of blood ALT and AST in this model. Furthermore, it inhibited liver cell necrosis and inflammatory cell infiltration.

(B6) Guinea-pig model of delayed asthma

[Method] Male Hartley guinea pigs were sensitized intraperitoneally with ovalbumin/Freund's complete adjuvant. On day 23 after immunization, the animals were caused to inhale a mist of ovalbumin solution from a sonic nebulizer for 3 minutes to elicit an asthmatic response. The drug was administered orally 1 hour before exposure to the antigen. The special airway resistance (sRaw) after exposure to the antigen was measured and the case in which the sRaw value had increased two-fold or more from the pre-exposure baseline in 4~8 hours was regarded as a case of delayed asthma.
[Results] The compound of this invention inhibited the delayed asthmatic response appearing 4~8 hours after exposure to the antigen in this model.

(B7) Atopy

[Method 1] Arachidonic acid-induced ear edema model:

To the left auricle of Balb/c mice, 2 mg/ear of arachidonic acid was applied to induce a skin inflammation. After 1 hour, the thickness of the auricle was measured with a peacock dial thickness gage and used as an indicator of skin inflammation. The drug was administered orally 30 minutes before application of arachidonic acid.
[Result-1] The compound of this invention significantly inhibited ear edema.

[Method 2] Spontaneous atopic dermatitis mode:

In the mouse model of NC/NGa dermatitis, the drug was administered orally once daily for 17 consecutive days.
[Result-2] The compound of this invention showed a significant disease state-ameliorating effect.

(B8) Inhibitory effect on antigen-induced eosinophil infiltration in guinea pigs

[Method] Male Hartley guinea pigs were sensitized by intravenous administration of guinea-pig anti-ovalbumin (OVA) antiserum. After 24 hours, an antigen challenge was performed by inhalation exposure to a nebulized 1% solution of OVA. At 24 hours after challenge with the antigen, the bronchial alveolar lavage (BAL) was collected and the number of eosinophils was determined using the eosinophil peroxidase activity in BAL as an indicator. The drug was administered orally 1 hour before the challenge. [Results] The compound of this invention inhibited the infiltration of eosinophils and other inflammatory cells into the bronchial alveola.
The release rates of the compound (I) of this invention and the free base (A) in Fluid 1 J.P. and the oral absorption rates thereof were measured. Samples: A suspension of polymorph A16 crystals in 0.5% methylcellulose (hereinafter referred to as Sample of Invention) and a suspension of free base (A) in 0.5% methylcellulose (hereinafter referred to as Control Sample)
Test sample size: 32 mg

(1) Release test

(1-1) Experimental conditions and procedure

Test method: JP XIII, Method 2 (paddle method)
Test fluid: J.P. Fluid 1
Test fluid volume: 900 ml
Rotational speed: 50 rpm
Temperature: 37°C

(1-2) Results

The release rate (%) in each time slot is shown below.

| | 5 min. | 10 min. | 15 min. | 20 min. | 30 min. | 45 min. | 60 min. | 90 min. | 120 min. |
|---|---|---|---|---|---|---|---|---|---|
| Control Sample | 11.8 | 13.6 | 14.7 | 15.4 | 16.6 | 17.6 | 18.3 | 19.4 | 20.3 |
| Sample of Invention | 92.6 | 94.2 | 94.2 | 94.4 | 94.3 | 94.3 | 94.5 | 94.4 | 94.5 |

(2) Oral absorption rate

(2-1) Method

Three male beagle dogs weighing about 10 kg were deprived of food from the day preceding the experiment day and the test sample was administered orally. Immediately after administration, 30 ml of water was given by oral gavage. After administration of each test sample, about 2.5 ml of blood was serially drawn from the antebrachial vein. The blood was treated with 25 μ l (25 U) of heparin J.P., the plasma was separated, and the plasma concentration of the drug was determined by HPLC. (2-2) Results

The plasma concentration (ng/ml) in each time slot is shown below.

| | 0.25 hr | 0.5 hr | 1 hr | 2 hr | 4 hr | 6 hr | 8 hr |
|---|---|---|---|---|---|---|---|
| Control Sample | 5.2 | 14.1 | 15.4 | 14.6 | 9.6 | 7.5 | 4.6 |
| Sample of Invention | 312.4 | 667.2 | 947.1 | 936.0 | 697.2 | 493.0 | 400.0 |

BEST MODE FOR CARRYING OUT THE INVENTION

The compound (I) of this invention can be synthesized not only by the process described in PCT laid-open Specification WO 96/01825 but also from synthetic free base (A) by the processes described in Preparations and Examples in this instant application.

For example, the following process can be mentioned as a typical process for synthesizing the A16 crystal.

The free base (A) of formula (A):

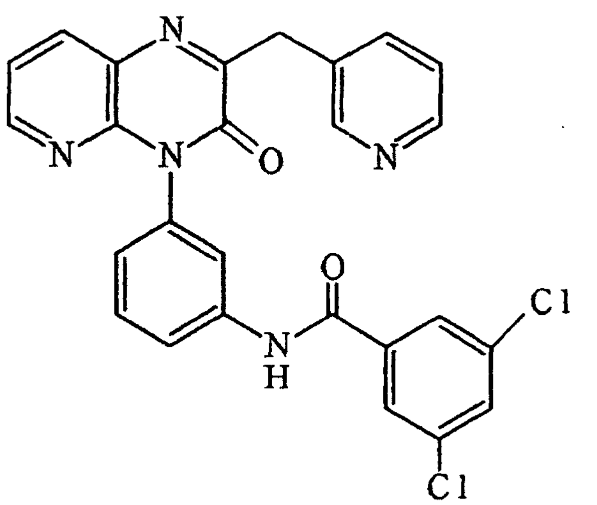

(A)

is suspended in methanol. After addition of methanesulfonic acid, the temperature is increased to dissolve the crystals and, then, decreased gradually to let crystals separate out. Thereafter, the system is cooled to near room temperature and stirred for ripening at that temperature. The crystals are then recovered by filtration to provide the objective polymorph A16 crystals.

To prove the usefulness of compound (I), studies concerning the solubility, stability and polymorphism were conducted.

(I) Studies on solubility and stability

For each sample (i.e. free base (A), compound (I)(A01 crystal), hydrochloride (B) and sulfate [hereinafter referred to as sulfate (C)]), the solubility values and the changes in crystallinity on compounding with water or ethanol taking the pharmaceutical procedure into consideration were studied.

(1) Solubility

The solubility in distilled water for injection and physiological saline solution was studied and the obtained results are as shown in Table 1.

Table 1

| Solubility values of each sample | | | | |
|---|---|---|---|---|
| | free base (A) | compound (I) (A01 crystal) | hydrochloride (B) | sulfate |
| Distilled water for injection | ≦ 0.001 mg/ml | 0.637 mg/ml | 0.588 mg/ml | 0.0295 mg/ml |
| Physiological saline solution | ≦ 0.001 mg/ml | 0.0439 mg/ml | 0.0446 mg/ml | 0.0389 mg/ml |

Compound (I) (A01 crystal) was found to have been improved in solubility in distilled water for injection and physiological saline solution as **compared with free base (A).**

(2) Changes in crystallinity upon compounding

For compound (I) and hydrochloride (B), changes in crystallinity upon compounding with ethanol were studied. The results are shown in Table 2.

Table 2

| Influence of compounding with ethanol on crystallinity | |
|---|---|
| | Ethanol |
| Compound (I) (A01 crystal) | No change (changed into in A16 crystal) |
| Hydrochloride (B) | Crystallinity decreased |

The crystallinity of hydrochloride was decreased when it was mixed with ethanol. A study of the solid state of this ethanol-compounded solid revealed that the residual amount was 91% at 70°C for 9 days. On the other hand, the ethanol-compounded sample of compound (I) showed no change in crystallinity.

(II) Study on polymorphism of the compound (I)

In the study on polymorphism of compound (I), six kinds of polymorphs (A01, A09, A16, A20, A30 and A43 crystals) were found. For each of those polymorphs, hygroscopicity, stability in solid state, solubility and changes in crystallinity on compounding with various solvents were studied. However, the A30 crystal was not studied since it contains ethanol.

(1) Hygroscopicity

The A09 andA43 crystals were highly hygroscopic . On the other hand, it was appeared that the A01, A16 and A20 crystals were less hygroscopic.

(2) Stability in solid state

The results of solid state stability are presented in Table 3.

Table 3

| Stability in solid state | | | | |
|---|---|---|---|---|
| | | A01 crystal | A16 crystal | A20 crystal |
| Initial | Appearance | Yellowish white crystalline powder | Pale yellowish white white crystalline powder | Pale yellowish white white crystalline powder |
| | Water content | 4.42 | 1.75 | 0.41 |
| | % residue | 100.0 | 100.0 | 100.0 |
| 70°C, 9 days | Appearance | Yellowish white crystalline powder | Pale yellowish white crystalline powder | Pale yellowish white crystalline powder |
| | Crystal form | Unchanged | Unchanged | Unchanged |
| | Water content | 4.23 | 1.68 | 0.30 |
| | % Residue | 97.7 | 100.3 | 99.6 |
| 70°C, 75% RH, 9 days | Appearance | Yellowish white crystalline powder powder | Pale yellowish white crystalline powder | Pale yellowish white crystalline powder |
| | Crystal form | Unchanged | Unchanged | Unchanged |
| | Water content | 4.76 | 5.09 | 1.22 |
| | % Residue | 99.9 | 101.6 | 101.0 |

The A01 crystal showed a slight decrease in percent residue after storage at 70°C for 9 days. On the other hand, both the A16 and A20 crystals were found to be chemically stable against heat and humidity and they show no change in crystal form.

(3) Solubility

The solubility (room temperature, 30-min shaking) of the A16 and A20 crystals in distilled water for injection and physiological saline solution was determined (Table 4).

| Table 4 Solubility values of A01, A16 and A20 crystals in various solvents | | | |
|---|---|---|---|
| | A01 crystal | A16 crystal | A20 crystal |
| Distilled injection for injection | 0.637 mg/ml | 0.887 mg/ml | 0.00876 mg/ml |
| Physiological saline solution | 0.0439 mg/ml | 0.0643 mg/ml | 0.0645 mg/ml |

The A16 crystal showed the highest solubility in distilled water for injection.

These results indicate that the compound (I), particularly its A16 crystal, has been improved in solubility and stability as compared with free base (A) and hydrochloride (B). Thus, it is considered that the A16 crystal is advantageous as the bulk substance for pharmaceutical production.

Finally, this invention provides the compound (I) representing improvements in the low solubility and stability of free base (A) and hydrochloride (B) and, in addition, the A16 crystal of compound (I) which shows no degradation in solid state and no change in crystallinity during compounding and represents an improvement in solubility as well.

The following examples illustrate this invention in further detail, it being to be understood that those are not intended to define the scope of the invention.

Preparation 1

2-Chloro-3-nitropyridine (50 g), N-acetyl-m-phenylenediamine (47.3 g) and anhydrous sodium carbonate (33.4 g) were suspended in dioxane (250 ml) and the suspension was refluxed with vigorous stirring. After the starting materials had dissolved once, the orange-colored objective crystals separated out gradually. The heating and stirring

was continued for 6 days, after which the reaction mixture was cooled and the objective crystalline product and the inorganic salt were separated by filtration. The crystals were washed with dioxane, dried and stirred together with water (1 L). Orange-colored crystals were recovered by filtration and rinsed with water until the washes had become neutral. The collected crystals were allowed to dry in the air to provide 2-(3-acetamidophenyl)amino-3-nitropyridine (52.9 g). NMR (DMSO-$d_6$, δ): 2.06 (3H, s), 6.99 (1H, dd, J=5 Hz, 8 Hz), 7.2-7.4 (3H, m) , 7.91 (1H, s) , 8.5-8.6 (2H, m), 9.93 (1H, s), 9.99 (1H, s)

Preparation 2

A mixture of 2-(3-acetamidophenyl)amino-3-nitropyridine (20 g) and 4N-hydrochloric acid (200 ml) was heated at 90°C with stirring for 2 hours. This reaction mixture was cooled and added portionwise to an aqueous solution of sodium hydrogen carbonate (68 g). The crystals which had separated out were recovered by filtration, rinsed with water and allowed to dry in the air to provide
2-(3-aminophenyl)amino-3-nitropyridine (17.8 g). NMR ($CDCl_3$, δ) : 3.72 (2H, s), 6.50 (1H,m), 6.80 (1H, m), 6.96 (1H, m), 7.12 (2H, m), 8.48 (2H, m) , 10.04 (1H, s)

Preparation 3

2-(3-Aminophenyl)amino-3-nitropyridine (17.8 g) was dissolved in dioxane (180 ml) under heating. After triethylamine (10.8 ml) was further added, a solution of 3,5-dichlorobenzoyl chloride (16.2 g) in dioxane (20 ml) was added dropwise with constant stirring. After completion of dropwise addition, the mixture was further stirred at room temperature for 1 hour. This reaction mixture was diluted with water and the resulting crystals were recovered by filtration. The crystals were rinsed with water and methanol and allowed to dry in the air overnight to provide crystals of 2-(3-(3,5-dichlorobenzoylamino)phenylamino)-3-nitropyridine (27.5 g).
NMR ($CDCl_3$, δ) : 7.00 (1H, m), 7.37 (1H, dd, J=8 Hz, 8 Hz), 7.45 (1H, m), 7.55 (1H, m), 7.88 (1H, m), 7.99 (2H, s), 8.10 (1H, m), 8.53 (2H, m), 9.98 (1H, s), 10.45 (1H, s)
m.p. 237-242°C

Preparation 4

A mixture of 2-(3-(3,5-dichlorobenzoyl)amino)phenylamino-3-nitropyridine (25 g), acetic acid (99.4 ml) and ethanol (300 ml) was heated with stirring. When the oil bath temperature had reached 50°C, iron power (17.3 g) was added, followed by further warming. Refluxing began after about 20 minutes, accompanied by separation of white crystals of iron acetate. The refluxing was continued for 2 hours, after which the reaction mixture was cooled and the objective crystals and the inorganic salt were recovered by filtration. This mixture was extracted with N,N-dimethylformamide (DMF) and the insoluble inorganic salt was filtered off. The DMF solution thus obtained was diluted with water, whereupon white crystals separated out. This crystal crop was harvested by filtration, rinsed with water and allowed to dry in the air overnight to provide crystals of
3-amino-2-(3-(3,5-dichlorobenzoylamino)phenylamino)pyridine (19.6 g).
NMR (DMSO-$d_6$, δ): 5.09 (2H, s), 6.62 (1H, dd, J=8 Hz, 5 Hz), 6.90 (1H, m) , 7.20 (2H, m) , 7.41 (1H, m), 7.50 (1H, m), 7.78 (1H, s), 7.86 (1H, s), 7.99 (2H, s), 8.05 (1H, s)

Preparation 5

To a suspension of 60% sodium hydride (1.49 g) in ether (1 ml) was dropwise added a mixed solution of N, N-dimethylglycine ethyl ester (7.9 ml) and 3-pyridinecarboxyaldehyde (2.0 g) in ether (4 ml) under stirring. When about one-fifth of the quantity had been added dropwise, the reaction began and the ether went on reflux. Then, cooling with iced water was started and the speed of dropwise addition was adjusted (over about 30 minutes) so that the reaction would proceed at an internal temperature of 20~30°C. After completion of dropwise addition, the reaction mixture was stirred at room temperature for 24 hours . When hydrogen gas had ceased to evolve, the reaction mixture was ice-cooled and ice (1 g) was further added. The exothermic reaction pushed the temperature up to 35°C and the system became solidified. After cooling to an internal temperature of 18°C, water (13 ml) and ether (20 ml) were added, followed by stirring. The ether layer was separated and the aqueous layer was extracted twice with 10 ml of ether each. The ether layers were combined, dried over anhydrous sodium sulfate and concentrated under reduced pressure (4.6 g).

The residue was subjected to distillation under reduced pressure. With a vacuum pump directly connected, the distillate up to a bath temperature of 140°C was collected to provide
3-(2-dimethylamino-2-(ethoxycarbonyl)ethenyl)pyr idine (3.1 g). (b.p. 110°C)
NMR ($CDCl_3$, δ): 1.25 (3H, t, J=7 Hz), 2.60 (6H, s), 4.22 (2H, q, J=7 Hz), 6.70 (1H, s), 7.40 (1H, m), 7.96 (1H, m), 8.45

(1H, m), 8.69 (1H, m)

Preparation 6

In 2N-sodium hydroxide (6 ml) was suspended 3-(2-dimethylamino-2-(ethoxycarbonyl)ethenyl)pyr idine (3.1 g), and the suspension was refluxed for 6 hours. This reaction mixture was washed with ethyl acetate (6 ml) and the aqueous layer was suction-filtered to remove both the oil and the precipitate which was formed in a minor amount. The aqueous solution was adjusted to pH 3.0 with concentrated hydrochloric acid (ca 1.5 ml) and allowed to stand under ice-cooling, whereupon the objective crystals separated out. The system was further allowed to stand overnight and the crystal crop was harvested by filtration, rinsed with water and allowed to dry in the air to provide 3-(3-pyridyl)pyruvic acid (1.1 g) as light-yellow crystals.
NMR ($D_2O$, δ) : 7.90 (1H, m), 8.40 (1H, m), 8.58 (2H, m)

Preparation 7

3-Amino-2-(3-(3,5-dichlorobenzoylamino)phen ylamino)pyridine (18.6 g) and 3-(3-pyridyl)pyruvic acid (8.23 g) were suspended in ethanol (370 ml) and the suspension was refluxed for 8 hours. This reaction mixture was cooled and the resulting crystals were recovered by filtration. The crystals thus obtained were rinsed with ethanol and dried to provide crystals of
4-(3-(3,5-dichlorobenzoylamino)phenyl)-2-(3-pyridyl)methyl-3-oxo-3,4-dihydroxyp yrido[2,3-b]pyrazine (21.2 g).
NMR (DMSO-$d_6$, δ): 4.27 (2H, s) , 7.12 (1H, d, J=8 Hz), 7.3-7.45 (2H, m), 7.56 (1H, t, J=8 Hz), 7.75-7.85 (3H, m) , 7.88 (1H, t, J=2 Hz), 7.98 (2H, d, J=2 Hz), 8.21 (1H, dd, J=2 Hz, 8 Hz), 8.41 (1H, d, J=5 Hz), 8.48 (1H, d, J=5 Hz), 8.60 (1H, d, J=2 Hz)

Example 1

In methanol was suspended 4-(3-(3,5-dichlorobenzoylamino)phenyl)-2-(3-pyri dyl)methyl-3-oxo-3,4-dihydroxypyrido[2,3-b]pyraz ine (12 g), and the suspension was warmed to about 60°C. After addition of methanesulfonic acid (2.29 g) , the system was cooled and the resulting crystals were recovered by filtration, rinsed with methanol and allowed to dry in the air overnight to provide 4-(3-(3,5-dichlorobenzoylamino)phenyl)-2-(3-pyridyl)methyl-3-oxo-3,4-dihy dropyrido[2,3-b]pyrazine methanesulfonate (13.3 g) as crystals.
NMR (DMSO-$d_6$, δ): 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7.89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

m.p. 158-166°C

Example 2

4-(3-(3,5-Dichlorobenzoylamino)phenyl)-2-(3 -pyridyl)methyl-3-oxo-3,4-dihydropyrido[2,3-b] py razine (20.0 g) was suspended in 98% methanol (MeOH) (200 ml). Then, methanesulfonic acid (MsOH) (3.83 g) was added and the temperature was increased to 60~65°C. After confirming dissolution of the crystals, the system was cooled gradually to let crystals separate out. The system was further cooled to 20~25°C and stirred at the same temperature for ripening. After ripening, the crystals were recovered by filtration to provide 4-(3-(3,5-dichlorobenzoylamino)phenyl)-2-(3-pyri dyl) methyl-3-oxo-3,4-dihydropyrido[2,3-b]pyrazin e methanesulfonate dihydrate (16.76 g) as light-yellow crystals.
The crystallographic morphology of the above crystals as determined by powder X-ray diffraction analysis was the polymorph A16.
NMR (DMSO-$d_6$, δ): 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1E, d, J=8 Hz), 7.89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Example 3

4-(3-(3,5-dichlorobenzoylamino)phenyl)-2-(3 -pyridyl)methyl-3-oxo-3,4-dihydropyrido[2,3-b]py razine (5.0 g) was suspended in 95% ethanol (EtOH) (50 ml). After the temperature was increased to 70~75°C, MsOH (0.96 g) was added. After confirming that the crystals had dissolved, the system was cooled gradually to let crystals separate out. The system was further cooled to 20~25°C and stirred at the same temperature for ripening. After ripening, the crystals

were recovered by filtration to provide 4-(3-(3,5-dichlorobenzoylamino)phenyl)-2-(3-pyri dyl)methyl-3-oxo-3,4-dihydropyrido[2,3-b]pyrazin e methanesulfonate (4.84 g) as light-yellow crystals.

The crystallographic morphology of the crystals thus obtained was confirmed to be the polymorph A30 by powder X-ray diffraction analysis. NMR (DMSO-$d_6$, δ): 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7.89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Example 4

4-(3-(3,5-Dichlorobenzoylamino)phenyl)-2-(3 -pyridyl)methyl-3-oxo-3,4-dihydropyrido[2,3-b]py razine methanesulfonate (0.5 g) (polymorph A16) was suspended in EtOH (5 ml). The temperature was then increased to 70~75°C and dissolution of the crystals was confirmed. This system was cooled gradually, whereupon crystals separated out. The system was further cooled to 20~25°C and stirred at the same temperature for ripening. After ripening, the crystals were recovered by filtration to provide 4-(3-(3,5-dichlorobenzoylamino)phenyl)-2-(3-pyridyl)methyl-3-oxo-3,4-dihydropyr ido[2,3-b]pyrazine methanesulfonate (0.43 g) as light-yellow crystals.

The crystallographic morphology of the crystals thus obtained was confirmed to be the polymorph A01 by powder X-ray diffraction analysis. NMR (DMSO-$d_6$, δ) : 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7,89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Example 5

4-(3-(3,5-Dichlorobenzoylamino)phenyl)-2-(3-pyridyl)methyl-3-oxo-3,4-dihydropyr ido[2,3-b]pyrazine methanesulfonate (1.0 g) (polymorph A30) was suspended in 95% EtOH (10 ml). This suspension was stirred as it was to ripen at 20~25°C. After ripening, the crystals were recovered by filtration to provide 4-(3-(3,5-dichlorobenzoylamino) phenyl)-2-(3-pyri dyl)methyl-3-oxo-3,4-dihydropyrido[2,3-b]pyrazin e methanesulfonate (0.86 g) as light-yellow crystals.

The crystallographic morphology of the crystals thus obtained was confirmed to be the polymorph A09 by powder X-ray diffraction analysis.
NMR (DMSO-$d_6$, δ): 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7.89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Example 6

4-(3-(3,5-Dichlorobenzoylamino)phenyl)-2-(3 -pyridyl)methyl-3-oxo-3,4-dihydropyrido[2,3-b]py razine methanesulfonate (3.5 g) (polymorph A30) was suspended in 86% EtOH (38.5 ml). The temperature was then increased to 70~75°C and dissolution of the crystals was confirmed. The system was then cooled gradually, whereupon crystals separated out. This system was further cooled to 20~25°C and allowed to ripen under stirring at the same temperature . After ripening, the crystals were recovered by filtration to provide 4-(3-(3,5-dichlorobenzoylamino)phenyl)-2-(3-pyri dyl) methyl-3-oxo-3,4-dihydropyrido[2,3-b]pyrazin e methanesulfonate (2.73 g) as light-yellow crystals.

The crystallographic morphology of the above crystals was confirmed to be the polymorph A43 by powder X-ray diffraction analysis.
NMR (DMSO-$d_6$, δ): 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7.89 (2H, m) , 7.98 (2H, m) , 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Example 7

4-(3-(3,5-Dichlorobenzoylamino)phenyl)-2-(3 -pyridyl)methyl-3-oxo-3,4-dihydropyrido[2,3-b]py razine methanesulfonate (1.0 g) (polymorph A30) was suspended in isopropyl alcohol (IPA) (20 ml). The temperature was then increased to 75~80°C and water (1.23 ml) was added. After confirming that the crystals had dissolved, 50 mg of seed crystals were added at 70~75°C. The system was then cooled gradually to let crystals separate out. The system was further cooled to 20~25°C and allowed to ripen under stirring at the same temperature. After ripening, the crystals were recovered by filtration to provide 4-(3-(3,5-dichlorobenzoylamino)phenyl)-2-(3-pyridyl)methyl-3-oxo-3,4-dihydropyr ido [2,3-b]pyrazine methanesulfonate (4.84 g) as light-yellow crystals.

The crystallographic morphology of the above crystals was confirmed to be the polymorph A20 by powder X-ray diffraction analysis.
NMR (DMSO-$d_6$, δ) : 2.32 (3H, s), 4.47 (2H, s), 7.10 (1H, d, J=8 Hz), 7.41 (1H, dd, J=8 Hz, 5 Hz), 7.57 (1H, dd, J=8 Hz, 8 Hz), 7.77 (1H, d, J=8 Hz), 7.89 (2H, m), 7.98 (2H, m), 8.01 (1H, dd, J=8 Hz, 5 Hz), 8.15 (1H, d, J=8 Hz), 8.43 (1H, d, J=5 Hz), 8.53 (1H, d, J=8 Hz), 8.83 (1H, d, J=5 Hz), 8.93 (1H, d, J=2 Hz), 10.64 (1H, s)

Reference Example 1

4-(3-(3,5-Dichlorobenzoylamino)phenyl)-2-(3 -pyridyl)methyl-3-oxo-3,4-dihydropyrido[2,3-b]py razine (5 g) was suspended in methanol (50 ml) followed by addition of sulfuric acid (0.98 g) . The mixture was warmed to about 60°C for dissolving . The solution was allowed to stand overnight and the resulting crystals were recovered by filtration, rinsed with methanol and dried to provide 4-(3-(3,5-dichlorobenzoylamino)phenyl)-2-(3-pyri dyl)methyl-3-oxo-3,4-dihydropyrido[2,3-b]pyrazin e sulfate (5.15 g).
m.p. 215-221°C
NMR (DMSO-$d_6$, δ): 4.45 (2H, m) , 7.10 (1H, d, J=8 Hz), 7.40 (1H, dd, J=8 Hz, 5 Hz), 7.55 (1H, dd, J=8 Hz, 8 Hz), 7.77 (1H, d, J=8 Hz), 7.89 (1H, m) , 7.99 (3H, m), 8.17 (1H, d, J=8 Hz), 8.43 (1H, m) , 8.49 (1H, d, J=8 Hz), 8.80 (1H, d, J=5 Hz), 8.90 (1H, s), 10.63 (1H, s)

BRIEF DESCRIPTION OF THE DRAWINGS

Fig. 1 is a powder X-ray diffraction pattern of the polymorph A01 crystal of compound (I).
Fig. 2 is a powder X-ray diffraction pattern of the polymorph A09 crystal of compound (I).
Fig. 3 is a powder X-ray diffraction pattern of the polymorph A16 crystal of compound (I).
Fig. 4 is a powder X-ray diffraction pattern of the polymorph A20 crystal of compound (I).
Fig. 5 is a powder X-ray diffraction pattern of the polymorph A30 crystal of compound (I).
Fig. 6 is a powder X-ray diffraction pattern of the polymorph A43 crystal of compound (I).

## Claims

1. A compound of the following formula or a solvate thereof.

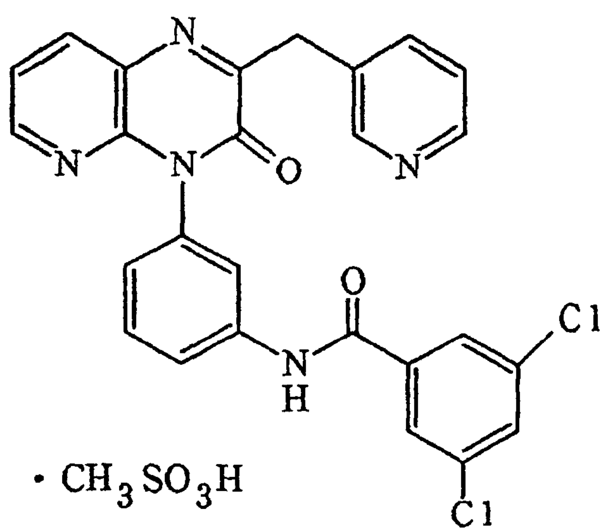

2. The compound according to Claim 1 which is of the following formula:

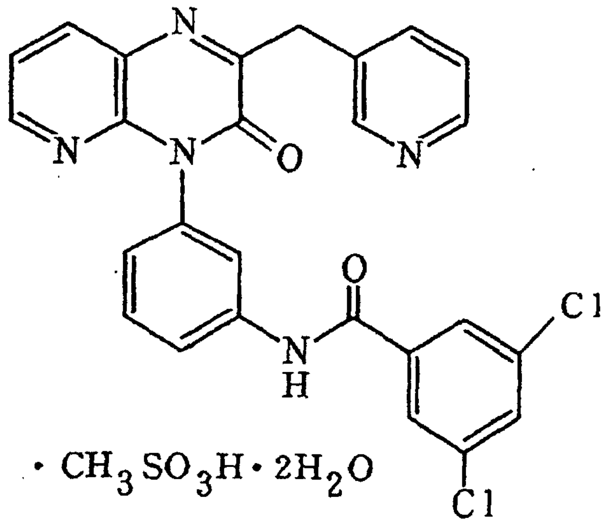

· $CH_3SO_3H$ · $2H_2O$

3. A crystal of the compound according to Claim 1 or 2 which shows substantially the following powder X-ray diffraction values:
Angles of diffraction:

2θ (° )=2.78, 5.59, 14.98, 16.73, 19.79, 24.61, 26.07,
2θ (° )=7.72, 10.07, 13.69, 15.23, 20.26, 21.03, 24.74, 25.17,
2θ (° )=2.86, 5.63, 20.86, 22.31, 23.88, 25.93,
2θ (° )=12.50, 19.28, 21.50, 23.16, 25.38,
2θ (° )=7.72, 10.03, 13.61, 20.05, 24.18, or
2θ (° )=7.08, 12.20, 21.33

4. The crystal claimed in Claim 3 which shows the following X-ray diffraction values.
Angles of diffraction:
2θ (° )=2.86, 5.63, 20.86, 22.31, 23.88, 25.93

5. Aprocess for producing the crystal claimed in Claim 4 characterized by its comprising suspending crystals of the compound of the following formula (A) in methanol, adding methanesulfonic acid thereto, increasing the temperature of the system to dissolve the crystals, cooling the system gradually to let crystals separate out, further cooling the system to the neighborhood of room temperature, and stirring the system at the same temperature for ripening.

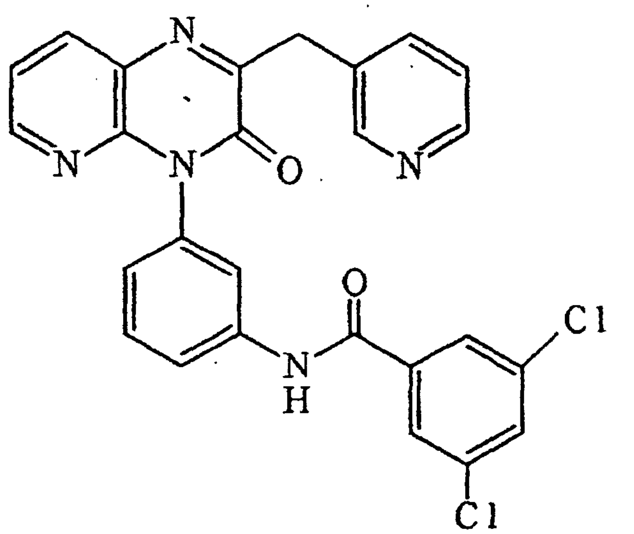

(A)

**6.** A therapeutic composition for hepatitis which comprises a compound having phosphodiesterase IV inhibitory activity, tumor necrosis factor production inhibitory activity, interferon production inhibitory activity or interleukin production inhibitory activity, use of said for the manufacture of a medicament for treating hepatitis, or a method for treatment of hepatitis which comprises administering said compound to a human being or an animal.

**7.** The therapeutic composition for hepatitis, the use of the compound for the manufacture of a medicament for treating hepatitis, or the method for treatment of hepatitis which comprises administering said compound to a human being or an animal, as claimed in Claim 6 wherein the hepatitis is viral, autoimmune or drug-induced hepatitis or hepatitis in the fulminating, acute or chronic stage of liver disorder associated with poisoning, ischemia, hyperthermia, hepatic infiltration of malignant cells, Wilson's disease or fatty liver.

**8.** A therapeutic composition for inhibiting the transition of various types of chronic hepatitis to cirrhosis or hepatocarcinoma, a therapeutic composition for primary biliary cirrhosis or a hepatoprotectant composition to be indicated postoperatively in liver transplantation or partial hepatectomy, which comprises a compound having phosphodiesterase IV inhibitory activity, tumor necrosis factor production inhibitory activity, interferon production inhibitory activity or interleukin production inhibitory activity.

**9.** Use of a compound having phosphodiesterase IV inhibitory activity, tumor necrosis factor production inhibitory activity, interferon production inhibitory activity or interleukin production inhibitory activity for the manufacture of a medicament for inhibiting the transition of various types of chronic hepatitis to cirrhosis or hepatocarcinoma, or for treating primary biliary cirrhosis, for useful as hepatoprotectant to be indicated postoperatively in liver transplantation or partial hepatectomy.

**10.** A method for inhibiting the transition of various types of chronic hepatitis to cirrhosis or hepatocarcinoma, for treating primary biliary cirrhosis, or for protecting hepatocytes to be indicated postoperatively in liver transplantation or partial hepatectomy, which comprises administering a compound having phosphodiesterase IV inhibitory activity, tumor necrosis factor production inhibitory activity, interferon production inhibitory activity or interleukin production inhibitory activity, to a human being or an animal.

**11.** A therapeutic composition for trachoma, allergic (inflammatory and atopic) conjunctivitis, glaucoma, ophthalmitis associated with herpesvirus or HIV infection; uveitis, AIDS, asthma, chronic obstructive pulmonary disease or atopic dermatitis, which comprises a compound having phosphodiesterase IV inhibitory activity, tumor necrosis factor production inhibitory activity, interferon production inhibitory activity or interleukin production inhibitory activity, use of said compound for the manufacture of a medicament for treating said diseases, or a method for treatment of said diseases which comprises administering said compound to a human being or an animal.

**12.** The therapeutic composition claimed in any of Claims 6-8 and 11, the use of the compound for the manufacture of a medicament for treating said diseases claimed in any of Claims 6-7, 9 and 11, or the method for treatment of said diseases which comprises of administering the compound to a human being or an animal claimed in any of Claims 6-7, 10 and 11, wherein the compound having phosphodiesterase IV inhibitory activity, tumor necrosis factor production inhibitory activity, interferon production inhibitory activity or interleukin production inhibitory activity is the following compound. Formula:

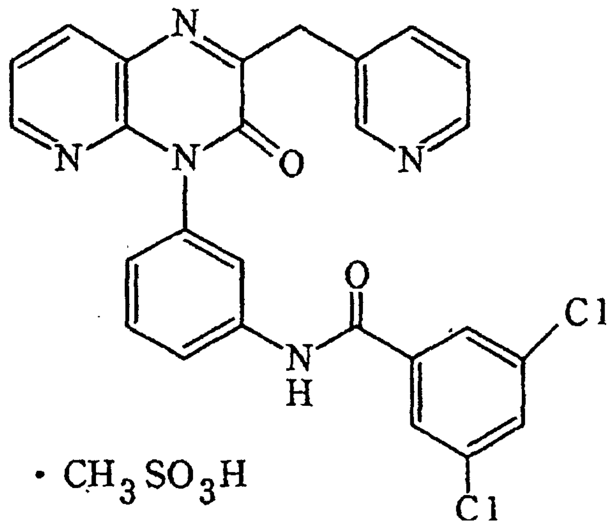

• $CH_3SO_3H$

or Formula:

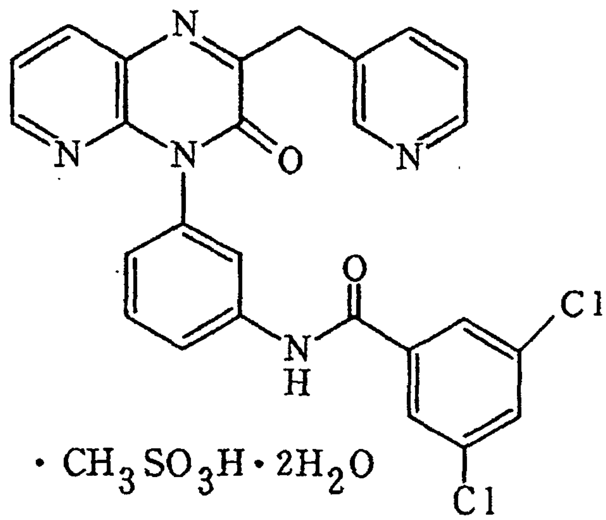

• $CH_3SO_3H \cdot 2H_2O$

**13.** The therapeutic composition claimed in any of Claims 6-8 and 11, the use of the compound for the manufacture of a medicament for treating said diseases claimed in any of Claims 6-7, 9 and 11, or the method for treatment of said diseases which comprises administering the compound to a human being or an animal claimed in any of Claims 6-7, 10 and 11, wherein the compound having phosphodiesterase IV inhibitory activity, tumor necrosis factor production inhibitory activity, interferon production inhibitory activity or interleukin production inhibitory activity is any of the compounds described in the following United States Patents.
3896000, 4356256, 5491147, 5580888, 5591776,
5622977, 5674880, 5686434, 5693659, 5710160,
5710170, 5712282, 5728712, 5744473, 5747506,
5753666, 5773467, 5776958, 5780667, 5786354,
5787215, 5792774, 5798373, 5804588, 5834485,
5849770, 5859008, 5859034, 5866593, 5889014,
5891878, 5891896, 5919801, 5922557, 5922740,
5922751, 5925636 and 5935977

**14.** The therapeutic composition claimed in any of Claims 6-8 and 11, the use of the compound for the manufacture of a medicament for treating said diseases claimed in any of Claims 6-7, 9 and 11, or the method for treatment of

said diseases which comprises administering the compound to a human being or an animal claimed in any of Claims 6-7, 10 and 11, wherein the compound having phosphodiesterase IV inhibitory activity, tumor necrosis factor production inhibitory activity, interferon production inhibitory activity or interleukin production inhibitory activity is any of the following compounds.
Arofylline, Atizoram, AWD 12281, BAY 198004, CDC 801, CDP 840, CI 1018, Cipamfylline, CP 146523, CP 166907, CP 220629, CP 293121, CP 353164, CP 77059, CP 80633, CT 1579, CT 1786, D 22888, D 4396, D 4418, DWP 205297, Filaminast (PDA641), GW 3600, KF 19514, L 0066, LAS 31025, LAS 32688, LAS 33774, MKS 213492, NCS 613, ORG 10325, ORG 30029, ORG 9731, PDB 093, PDE-IV inhibitors, Zambon, Piclamilast (RP 73401), Roflumilast, Rolipram, RPR 116474, RPR 132294, RPR 132703, SB 207499, SDZISQ 844, SelCIDs, SH636, T 440, Tolafentrine, WAY 122331, WAY 127093B, WIN 65579, YM 976 and Zardaverine

Fig. 1

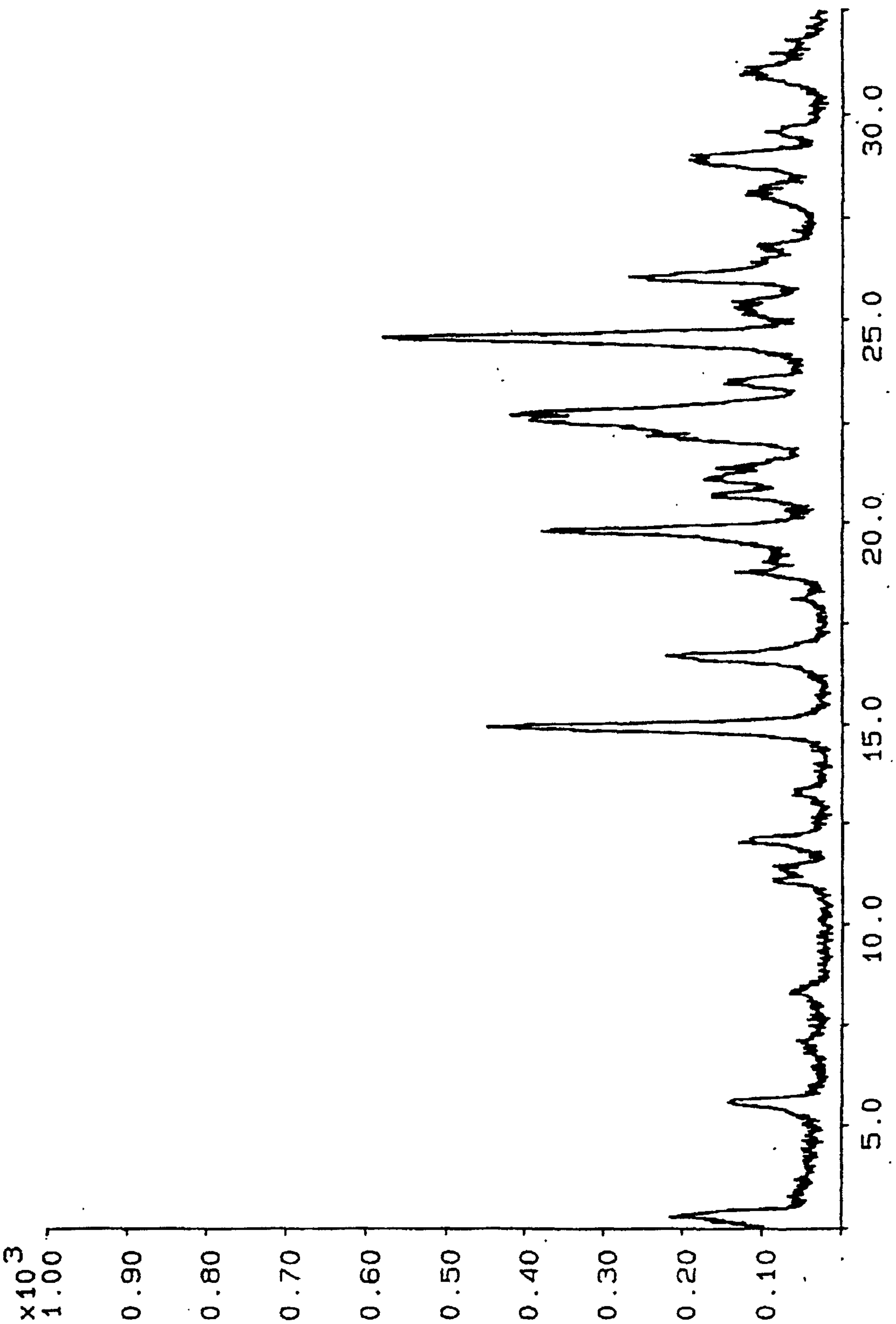
x10 3
1.00
0.90
0.80
0.70
0.60
0.50
0.40
0.30
0.20
0.10
5.0
10.0
15.0
20.0
25.0
30.0

Fig. 2

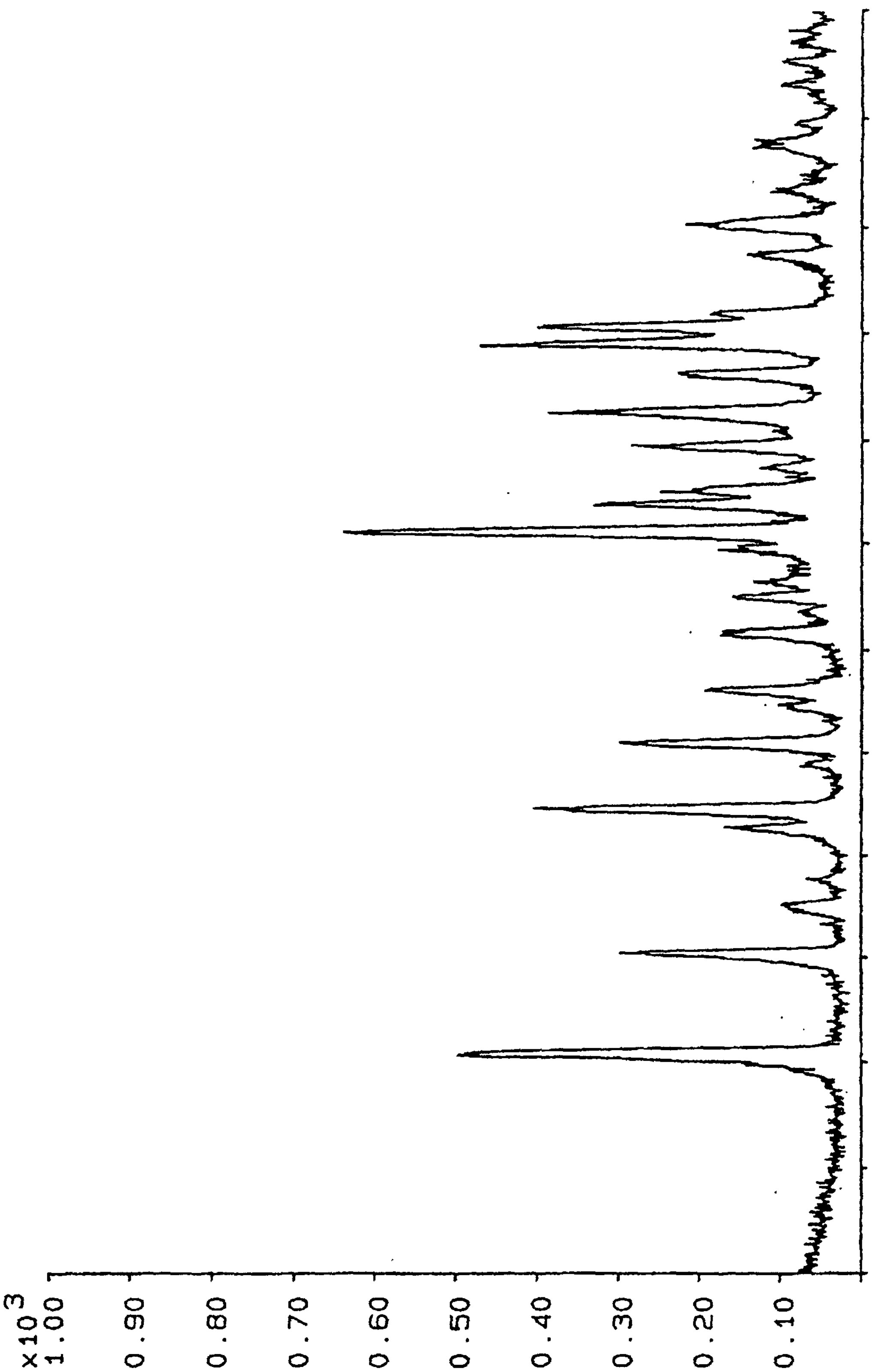
x10^3
1.00
0.90
0.80
0.70
0.60
0.50
0.40
0.30
0.20
0.10
5.0
10.0
15.0
20.0
25.0
30.0

Fig. 3

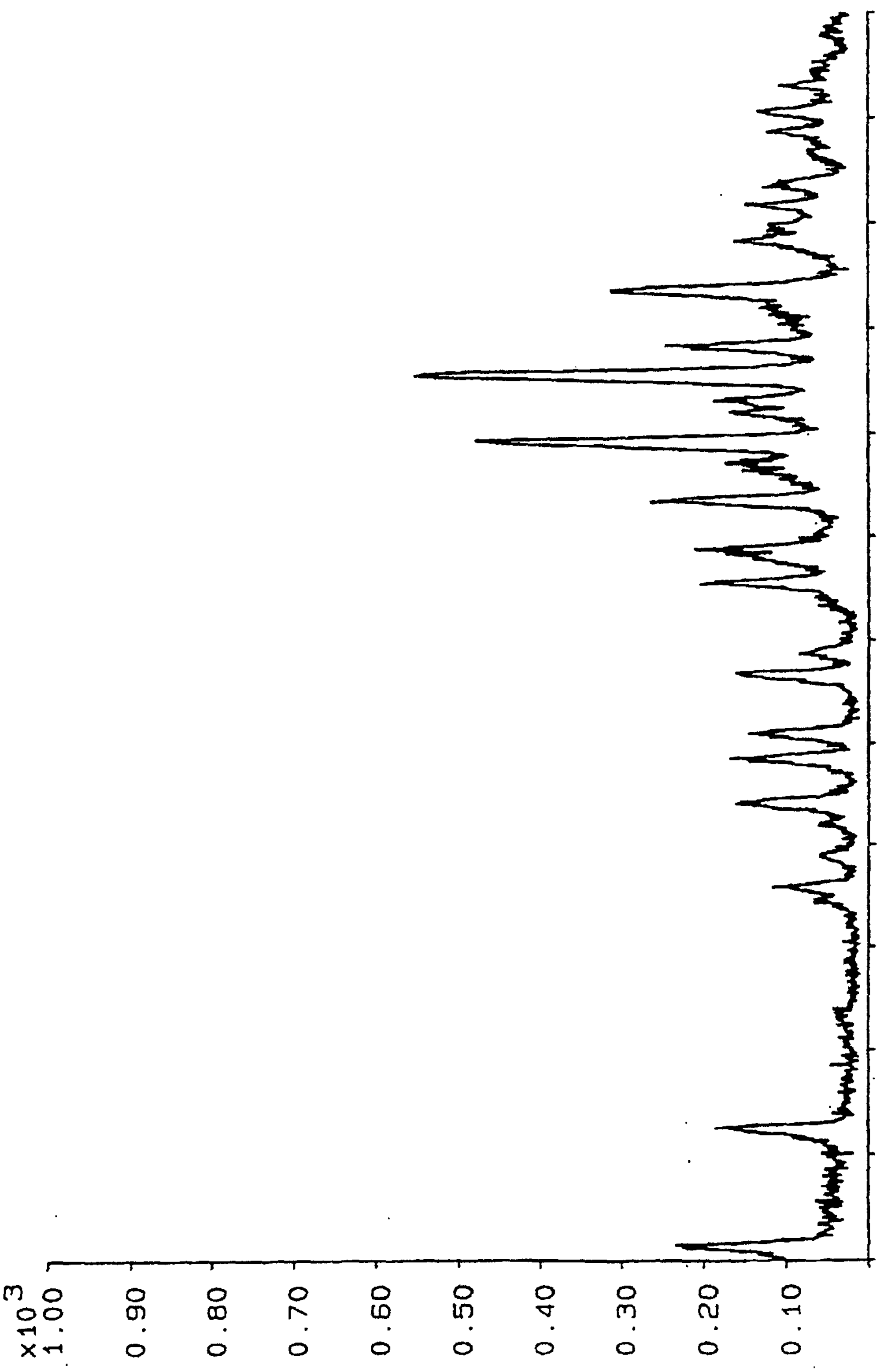
x10^3
1.00
0.90
0.80
0.70
0.60
0.50
0.40
0.30
0.20
0.10
5.0
10.0
15.0
20.0
25.0
30.0

Fig. 4

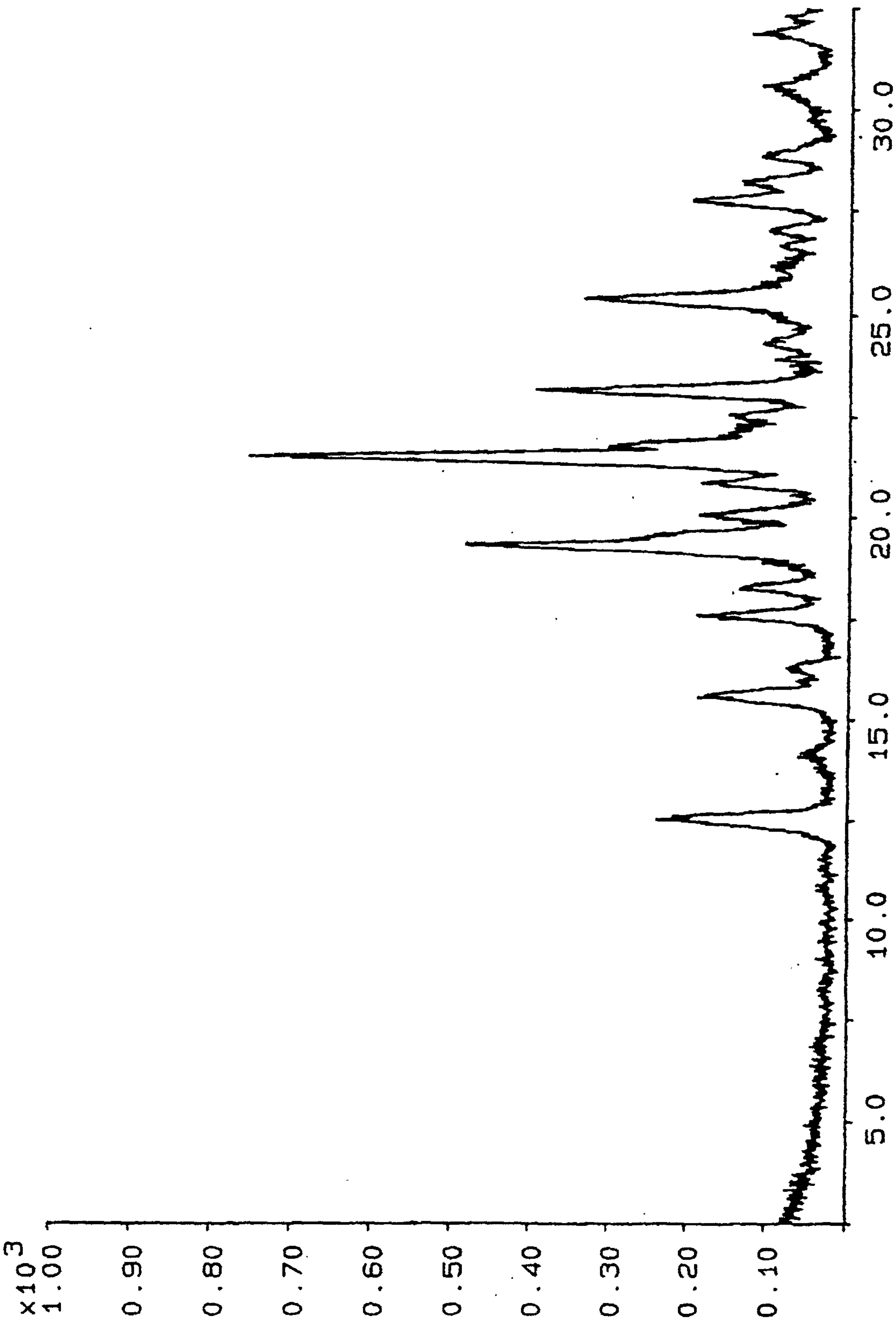
x10
3
1.00
0.90
0.80
0.70
0.60
0.50
0.40
0.30
0.20
0.10
5.0
10.0
15.0
20.0
25.0
30.0

Fig. 5

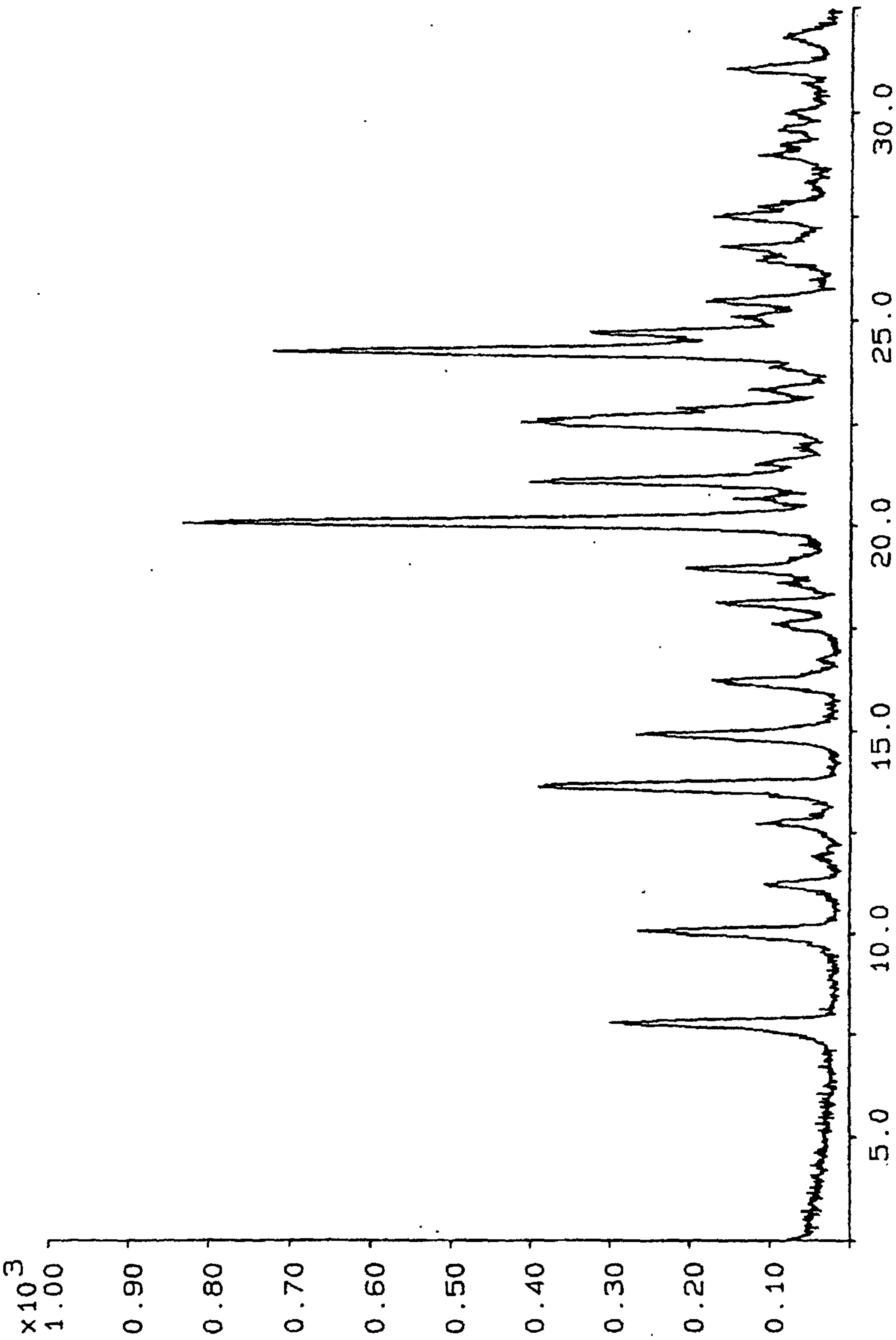
x10^3
1.00
0.90
0.80
0.70
0.60
0.50
0.40
0.30
0.20
0.10
5.0
10.0
15.0
20.0
25.0
30.0

Fig. 6

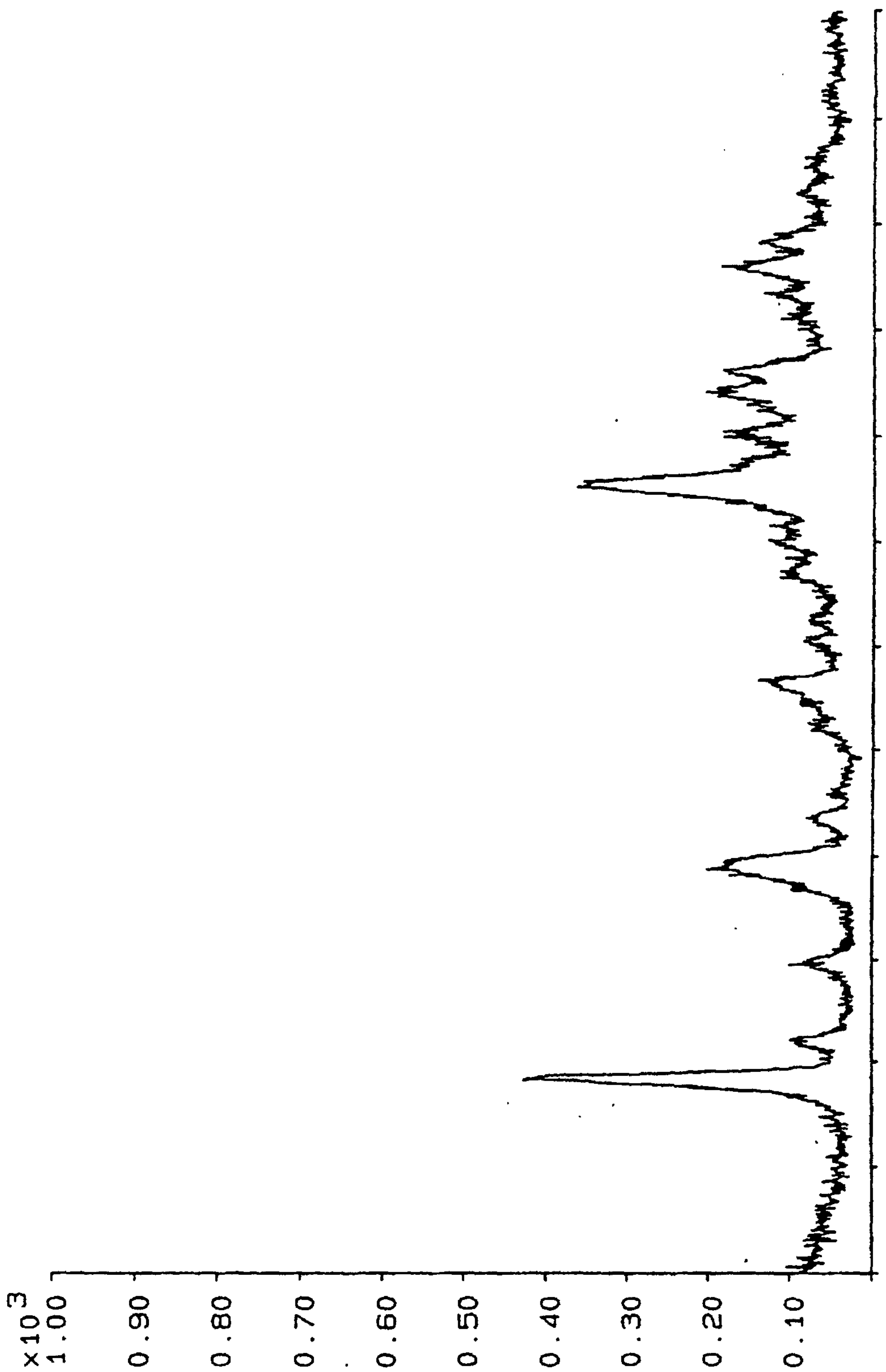
×10³
1.00
0.90
0.80
0.70
0.60
0.50
0.40
0.30
0.20
0.10
5.0
10.0
15.0
20.0
25.0
30.0

## INTERNATIONAL SEARCH REPORT

International application No.
PCT/JP99/05182

A. CLASSIFICATION OF SUBJECT MATTER
Int.Cl[6] C07D471/04, A61K31/4985, A61K45/00, A61P43/00, 1/16, 11/00,11/06, 27/02, 27/06, A61P27/14, 31/18, 37/08

According to International Patent Classification (IPC) or to both national classification and IPC

B. FIELDS SEARCHED

Minimum documentation searched (classification system followed by classification symbols)
Int.Cl[6] C07D471/04, A61K31/4985, A61K45/00, A61P43/00, 1/16, 11/00, 11/06, 27/02, 27/06, A61P27/14, 31/18, 37/08

Documentation searched other than minimum documentation to the extent that such documents are included in the fields searched

Electronic data base consulted during the international search (name of data base and, where practicable, search terms used)
CAPLUS, REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO, 9601825, A (Fujisawa Pharmaceutical Co., Ltd.), 25 January, 1996 (25.01.96) & EP, 770079, A & EP, 920867, A & JP, 10502630 | 1-9,11,12 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 December, 1999 (21.12.99) | 18 January, 2000 (18.01.00) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/05182

**Box I Observations where certain claims were found unsearchable (Continuation of item 1 of first sheet)**

This international search report has not been established in respect of certain claims under Article 17(2)(a) for the following reasons:

1. ☒ Claims Nos.: 10
because they relate to subject matter not required to be searched by this Authority, namely:

The subject matter of claim 10 relates to a method for treatment of the human body by therapy, which does not require an international search report by this International Search Authority in accordance with PCT Article 17(2)(a)(i) and Rule 39.1(iv).

2. ☒ Claims Nos.: 13, 14
because they relate to parts of the international application that do not comply with the prescribed requirements to such an extent that no meaningful international search can be carried out, specifically:

Since claims 13 and 14 describe compounds in terms of numbers of patent documents cited or names not based on the common nomenclature of chemistry, what the compounds set forth in these claims specifically refer to cannot be understood even if the disclosure of the description and the common general technical knowledge are taken into consideration.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

See Extra Sheet.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP99/05182

Continuation of Box II of continuation of first sheet(1)

(1) Claim 1 to 5 of the application concerned describe the pyridopyrazine compound as set forth in claim 1; solvates and crystals thereof; and processes for the preparation of the crystals.

(2) Claims 6 to 14 of the application concerned describe drugs such as hepatitis remedies, inhibitors against the transition of various chronic hepatitides into hepatic cirrhosis or liver cancer, remedies for primary biliary cirrhosis, liver protecting agents for use after the transplantation or partial excision of liver, trachoma remedies and allergic conjunctivitis remedies (hereinafter referred to merely as "hepatitis remedies and so on"), containing compounds exhibiting phosphodiesterase IV inhabiting activities, tumor necrosis factor production inhabiting activities, interferon production inhibiting activities or interleukin production inhibiting activities (hereinafter referred to merely as phosphodiesterase IV inhibiting activities and so on)"; use of such compounds in preparing drugs including hepatitis remedies and so on; and methods for treatment of hepatitis and so on by administering the compounds to human being and so on.

(3) Thus, a group of inventions of the item (2) is characterized by using, as hepatitis remedies and so on, compounds which are not limited to the pyridopyrazine compounds of invention of the item (1) but may be general compounds exhibiting phosphodiesterase IV inhibiting activities and so on, and is therefore not considered as forming a single general inventive concept together with a group of inventions of the item (1) characterized by providing novel pyridopyrazine compounds.